# EUROPÄISCHE PATENTSCHRIFT

(11) **EP 1 610 129 B1**
(45) Veröffentlichungstag und Bekanntmachung des Hinweises auf die Patenterteilung: **24.10.2012**
(21) Anmeldenummer: 05013260.4
(22) Anmeldetag: 20.06.2005
(51) Int. Cl.: G01N 33/543, G01N 33/531, B05D 1/34, G01N 35/10, B01L 99/00, B01J 19/00

(54) **Verfahren und Vorrichtung zur Herstellung von bindungsfähigen Reagenzträgern**
Process and apparatus for manufacturing affinity-binding supports
Procédé et dispositif de préparation de substrates avec affinité pour un analyte

(30) Priorität: 21.06.2004 DE 102004029909
(43) Veröffentlichungstag der Anmeldung: 28.12.2005
(73) Patentinhaber: Roche Diagnostics GmbH, 68305 Mannheim (DE); F. Hoffmann-La Roche AG, 4070 Basel (CH)
(72) Erfinder: Hornauer, Hans, 82380 Peissenberg (DE)
(74) Vertreter: Tiesmeyer, Johannes

(56) Entgegenhaltungen:
- EP-A- 0 833 158
- US-A- 5 183 508
- US-A- 5 378 638
- US-A- 5 547 702
- US-A1- 2004 018 635

## Beschreibung

Die Erfindung betrifft ein Verfahren zur Herstellung von bindungsfähigen und zur Bestimmung von Analyten in Flüssigkeiten geeigneten Reagenzträgern nach dem Oberbegriff des Anspruchs 1 und eine Präparationsvorrichtung zur Durchführung des Verfahrens gemäß Anspruch 5.

Die Erfindung befasst sich mit der kostengünstigen Herstellung bzw. Präparation von Reagenzträgern zur qualitativen oder quantitativen Bestimmung von Analyten in Flüssigkeiten, insbesondere in Körperflüssigkeiten. Solche an sich bekannten Reagenzträger sind in der Lage, in einer Reaktion ("Assay") Analytmoleküle aus der Probenflüssigkeit an einer Trägeroberfläche zu binden, wobei die Bindungsereignisse messtechnisch nachweisbar sind, z.B. durch optischen Nachweis von Fluoreszenzereignissen. Reagenzträger der hier betrachteten Art können nach dem Verfahren der vorliegenden Erfindung z.B. mit Mikroarray-Strukturen präpariert werden, etwa um einen Biochip für den ortsselektiven Nachweis von Bindungsreaktionen herzustellen.

Die Erfindung ist aber nicht nur auf die Herstellung von Biochips mit Mikroarrays beschränkt, sondern eignet sich auch zur Herstellung flächig beschichteter Träger.

Bei der Herstellung von Reagenzträgern der hier betrachteten Art wird üblicherweise ein jeweiliger Oberflächenbereich der vorweg bereitgestellten Reagenzträgerkörper mit einer Flüssigkeit oder ggf. Flüssigkeiten in wohl definierter zeitliche Abfolge in Kontakt gebracht. Hierzu werden Präparationsvorrichtungen eingesetzt, welche technische Einrichtungen zur volumenkontrollierten Zugabe von Flüssigkeiten aufweisen. Ferner können Präparationsvorrichtungen mit Einrichtungen zum Entfernen vorher zugeführter Flüssigkeiten oder zum Entfernen schwach gebundener oder eingeschlossener Moleküle oder Partikel von der Trägerkörperoberfläche zum Einsatz kommen. Als Behandlungseinrichtungen kommen überdies Wascheinrichtungen, Trocknereinrichtungen u. dgl. in Frage.

Die Präparationsvorrichtungen umfassen üblicherweise eine Steuereinrichtung zur Steuerung vorbestimmter zeitlicher Abfolgen von Prozessschritten. Die Abfolge von Prozessschritten kann durch Wartezeiten unterbrochen sein, die erforderlich sind, um Reaktionen ablaufen zu lassen. Die Reagenzträgerkörper können zu diesem Zweck zu Zwischenlagerungspositionen in der Präparationsvorrichtung oder außerhalb der Präparationsvorrichtung transportiert werden. An den Zwischenlagerungspositionen können Thermostatisierungseinrichtungen, Schüttler usw. für die Reagenzträgerkörper vorgesehen sein.

Bei konventionellen Systemen zur Herstellung bzw. Präparation von Reagenzträgern wird ein jeweiliger Reagenzträgerkörper zu einer Behandlungseinrichtung hin transportiert und dort im Ruhezustand einem Behandlungsschritt unterzogen. Nach der Behandlung wird der Reagenzträgerkörper dann aus der Behandlungszone entfernt und ggf. einer weiteren Behandlungseinrichtung zugeführt usw. Diese konventionelle Vorgehensweise erlaubt im Allgemeinen nur eine relativ geringe Ausbringungsmenge an präparierten Reagenzträgern pro Zeiteinheit. Es sind auch bereits Vorschläge gemacht worden, mehrere Behandlungseinrichtungen in einer Präparationsvorrichtung zu parallelisieren. Die Behandlungseinrichtungen sind dabei in mehrfacher Ausführung vorgesehen, so dass sie Reagenzträgerkörper gruppenweise parallel behandeln können. Wenngleich auf diese Weise ein höherer Durchsatz bei der Präparation von Reagenzträgern möglich ist, so tritt jedoch das Problem auf, dass aufgrund von Exemplarstreuungen der Behandlungseinrichtungen unterschiedliche Ergebnisse bei der Behandlung der einzelnen Reagenzträgerkörper auftreten. Es ist daher ein erhöhter Aufwand notwendig, um hinreichend gleiche Funktionalität der parallelen Behandlungskanäle untereinander zu gewährleisten und durch Prüfung zu verifizieren.

In manchen Fällen ist eine Parallelisierung von Präparationsschritten an mehreren Reagenzträgerkörpern nicht praktikabel. Ein Beispiel hierfür ist die ortspräzise Ablage von Flüssigkeitströpfchen bei der Herstellung von Mikroarrays. Dabei sollen unterschiedliche Bereiche der Oberfläche eines Reagenzträgerkörpers mit unterschiedlichen Flüssigkeiten beaufschlagt werden, um so im späteren Assay innerhalb ein und desselben Ablaufs mehrere verschiedene Bestandteile der Probenflüssigkeit gleichzeitig bestimmen zu können, weil die unterschiedlichen Bereiche unterschiedliche spezifische Reaktionen mit Bestandteilen der Probenflüssigkeit eingehen. In der Veröffentlichung "Kuhn et al / BIOforum Int., S. 30 ff, 2000-1" ist eine Hochdurchsatzanlage mit Ausbringungsmengen bis zu 5000 Trägern pro Tag beschrieben.

Es ist auch bereits bekannt, bei der Teststreifenherstellung das Trägerpapier von einer Vorratsrolle abzuwickeln und das Streifenband in einem kontinuierlichen Vorgang durch ein Tauchbad zu leiten bzw. mit Testsubstanzen zu besprühen. An den Vorgang der Beschichtung schließt sich jedoch ein sehr aufwendiger Vorgang der Device-Herstellung an, indem das Streifenband in Form geschnitten und die vereinzelten Papierstreifen aufwendig in einzelne Halterungen eingepasst und zu handhabbaren Elementen zusammengefügt werden.

Die US-A-5 378 638 offenbart einen Produktionsprozess für ein Analyseelement mit einer Trägerschicht, auf die eine Reagenzregion mit einem mittels Tintenstrahldruckverfahren aufgebrachten Reagenzmuster aufgebracht wird, wobei das Aufbringverfahren die Verwendung eines Tintenstrahldüsenkopfes vorsieht, der mittels Relativverschiebung zur Trägerschicht diskrete Reagenzspots auf letztere aufsprüht. Als Reagenzträgerkörper werden bei dem Verfahren der US-A-5 378 638 Kunststofffilmelemente verwendet, welche eine für ein derartiges Tintenstrahldruckverfahren geeignete ebene Oberfläche bieten.

Die EP 0 833 158 A1 beschreibt einen Produktionsprozess für eine in einzelne identische Teststreifen unterteilbare Teststreifenbahn, wobei ein poröses Trägermaterial mit einem speziellen Analytbindereagenz beschichtet wird, welches durch einen Sprühkopf aufgebracht wird, der sich relativ zum stationären Trägermaterial bewegt; alternativ kann der Sprühkopf fixiert sein und das Trägermaterial daran vorbeibewegt werden.

Der Erfindung liegt die Aufgabe zugrunde, ein Verfahren und eine Vorrichtung bereitzustellen, welche mit einfachen Mitteln die Herstellung von bindungsfähigen und zur Bestimmung von Analyten in Flüssigkeiten geeigneten Reagenzträgern unter Verwendung vorgefertigter, obenseitig wannenartig ausgehöhlter Reagenzträgerkörper mit sehr großer Ausbringleistung ermöglichen.

Das erfindungsgemäße Verfahren zur Herstellung von bindungsfähigen und zur Bestimmung von Analyten in Flüssigkeiten geeigneten Reagenzträgern verwendet als Reagenzträgerkörper (4) vorgefertigte Reaktionsgefäße, die obenseitig wannenartig ausgehöhlt sind und in einem kontinuierlichen Durchlauf durch die Behandlungszone (6; 6b) hindurchtransportiert werden, wobei der wenigstens eine Behandlungsschritt an den bewegten Reagenzträgerkörpern (4) erfolgt.

Bei der Behandlung des Reagenzträgerkörpers während seines Transports in der Präparationsvorrichtung entfällt das stationäre Positionieren der einzelnen Reagenzträgerkörper in der Behandlungszone der Behandlungseinrichtung. Die Reagenzträgerkörper erfahren somit bei ihrem kontinuierlichen Durchlauf durch die betreffende Behandlungszone einen Behandlungsschritt. Abbremszeiten und Beschleunigungszeiten für das Einbringen des Trägerkörpers in die Behandlungszone und für das Herausführen des Trägerkörpers aus der Behandlungszone werden somit obsolet, wodurch ein enormer Zeitgewinn bei der Serienpräparation der Reagenzträger erzielt wird.

Basisidee eines Aspektes der Erfindung ist es, die Prozessabläufe so zu gestalten, dass das bei den konventionellen Systemen verwirklichte Prinzip "Prozessausführung erfolgt im mechanischen Ruhezustand" bei möglichst vielen Behandlungsschritten wegfällt und dass stattdessen die Behandlungsschritte während der Transportbewegung der einzelnen aufeinander folgenden Reagenzträgerkörper stattfinden.

Zeitvorteile sind auch in dem Fall erzielbar, dass der wenigstens eine Behandlungsschritt während einer Bewegung der Behandlungseinrichtung relativ zum Reagenzträgerkörper in der Präparationsvorrichtung erfolgt. Eine derartige Prozessführung kann z.B. das gruppenweise Einbringen einer Vielzahl von Reagenzträgerkörpern in eine Behandlungszone umfassen, wobei die Behandlungseinrichtung dann über die Reagenzträgerkörper hinwegbewegt wird, um dabei den betreffenden Behandlungsschritt durchzuführen. Danach kann dann der automatische Abtransport der behandelten Reagenzträgerkörper und das Bereitstellen einer neuen Gruppe von Reagenzträgerkörpern in der Behandlungszone erfolgen.

Der wenigstens eine Behandlungsschritt am bewegten Reagenzträgerkörper erfolgt während seines Transports in der Präparationsvorrichtung, wobei zusätzlich die Behandlungseinrichtung im Bereich einer Behandlungszone eine Synchronbewegung oder eine Relativbewegung zu dem bewegten Reagenzträgerkörper ausführt.

Sofern zur Präparation der Reagenzträger mehrere schnell ablaufende Behandlungsschritte vorzunehmen sind, so sollten so viele dieser Schritte wie möglich bei bewegtem Reagenzträgerkörper und ggf. bei bewegter Behandlungseinrichtung durchgeführt werden, um möglichst wenige Start/Stopp-Vorgänge bzw. Stationierungs- und Positionierungsvorgänge ausführen zu müssen. In geeigneten Fällen kann es jedoch durchaus zweckmäßig sein, bei der Durchführung mehrerer Behandlungsschritte einige dieser Behandlungsschritte in konventioneller Weise durchzuführen, etwa nach dem oben bereits angesprochenen Prinzip der Parallelisierung einzelner Prozessschritte.

Die an den vorher bereitgestellten einzelnen Reagenzträgerkörpern vorzunehmenden Behandlungsschritte und die dazu zu verwendenden Behandlungseinrichtungen können vielfältiger Art sein. So kann ein erster Behandlungsschritt z.B. darin bestehen, dass eine jeweilige Reaktionsfläche der Reagenzträgerkörper mit einer Substanz, etwa einer Flüssigkeit, vollständig überschichtet wird, indem dem Reagenzträgerkörper eine dosierte Menge dieser Substanz mittels einer Dosiereinrichtung zugeführt wird.

Ein anderer Behandlungsschritt kann darin bestehen, dass auf einem jeweiligen Oberflächenbereich der Reagenzträgerkörper Flüssigkeitströpfchen an vorbestimmten Stellen abgelegt werden, um einen Mikroarray zu bilden. Die Abgabe der Flüssigkeitströpfchen erfolgt mit Tröpfchenerzeugem nach dem Inkjet-Prinzip, wie es von Tintenstrahldruckern her bekannt ist. Erfindungsgemäß bewegen sich der Reagenzträgerkörper und der Tröpfchenerzeuger relativ zueinander und werden anlässlich der Tropfenabgabe nicht angehalten.

Ein anderer Behandlungsschritt kann darin bestehen, dass Substanzen, etwa Flüssigkeiten, mittels einer Absaugeinrichtung von den Reagenzträgerkörpem entfernt werden.

Ein weiterer Behandlungsschritt kann z.B. im Austausch von Lösungen an Reagenzträgerkörpern bestehen. Dabei kann es sich z.B. um einen Spülschritt oder einen Waschschritt handeln. Auch in diesem Fall wird erfindungsgemäß eine Prozessführung unter weitgehender Vermeidung von Start/Stopp-Vorgängen in der Präparationsvorrichtung angestrebt.

Das Verfahren nach der Erfindung kann u.a. angewandt werden, um lokal begrenzte Festphasen für Bindungsassays zu erzeugen, wie dies z.B. in der EP 0 939 319 A2 oder in der EP 1 380 839 A1 vom Grundprinzip her offenbart ist. So kann das Verfahren nach der Erfindung zum Aufbringen einer mehrlagigen Beschichtung auf einen festen, nicht porösen Reagenzträgerkörper verwendet werden, wobei in aufeinander folgenden Behandlungsschritten am kontinuierlich bewegten Reagenzträgerkörper eine Vorbeschichtung auf ein Reagenzfeld des Reagenzträgerkörpers aufgebracht wird, der vorbeschichtete Reagenträgerkörper mit einer wässrigen Flüssigkeit gewaschen wird und eine zweite Beschichtung auf den vorbeschichteten Reagenträgerkörper in Form von räumlich begrenzten Flächen auf dem Reagenzfeld aufgebracht wird, wobei die zweite Beschichtung Rezeptormoleküle umfasst, die mit der Vorbeschichtung bindefähig sind.

Die Vorbeschichtung kann dabei vollflächig auf einen Teil oder auf die gesamte Fläche eines Reagenzfeides des festen Reagenträgerkörpers oder auch in Form von Spots aufgetragen werden. Bevorzugt wird die Vorbeschichtung vollflächig aufgetragen. Die Vorbeschichtung wird typischerweise aus einer wässrigen Lösung auf den Träger aufgebracht. Sie kann aus beliebigen Molekülen bestehen, welche die Bindung einer zweiten Beschichtung ermöglichen. Bevorzugt umfasst die Vorbeschichtung einen ersten Partner eines hochaffinen Bindepaares, wie z.B. Streptavidin, Avidin oder Biotin, sowie Analoga, Derivate oder Konjugate der vorstehenden Substanzen, oder Antikörper. Es können aber auch Moleküle als Vorbeschichtung aufgebracht werden, die für eine kovalente Bindung mit der zweiten Beschichtung vorgesehen sind, etwa Moleküle, die eine Amin-, eine Sulfit- oder eine Silylgruppe enthalten. Nach dem Waschen des vorbeschichteten Reagenträgerkörpers werden dann auf die Vorbeschichtung Rezeptormoleküle in wässriger Lösung in Form kleiner Tröpfchen aufgebracht. Die Rezeptormoleküle können aus der Lösung an die Vorbeschichtung diffundieren und an diese binden. Es sind somit Multianalyt-Träger mit Multianalyt-Beschichtung auf einer betreffenden Vorbeschichtung mit dem Verfahren nach der Erfindung herstellbar. Grundsätzlich sind unter Verwendung des Verfahrens nach der Erfindung Träger herstellbar, wie sie z.B. in der EP 0 939 319 A2 bzw. in der EP 1 380 839 A1 beschrieben sind.

So können unter Verwendung des Verfahrens nach der Erfindung z.B. auch Reagenzträger hergestellt werden, indem an den bewegten Reagenzträgerkörpern in aufeinander folgenden Schritten eine Rezeptormoleküle enthaltende Beschichtungslösung auf vorbestimmte begrenzte Bereiche (Spots) der Reagenzträgerkörper aufgebracht wird, die Rezeptormoleküle in den räumlich begrenzten Bereichen gebunden werden, die Beschichtungslösung getrocknet wird und eine Nachbeladelösung aufgebracht wird.

Diese Verfahrensschritte sind für die konventionelle Prozessführung in der EP 0 939 319 A2 und in der EP 1 380 839 A1 erläutert, deren Offenbarungsgehalt in die vorliegende Anmeldung einbezogen wird.

Das Verfahren nach der Erfindung kann somit angewandt werden, um streptavidinbeschichtete Träger, HBc-Antigen-Träger oder Multianalyt-Träger herzustellen bzw. zu präparieren. Damit sind die Anwendungsmöglichkeiten jedoch noch nicht erschöpft.

Zur Durchführung des Verfahrens nach der Erfindung wird eine Präparationsvorrichtung vorgeschlagen, die gekennzeichnet ist durch wenigstens eine Behandlungseinrichtung zur Behandlung von Reagenzträgerkörpem in Form von Reaktionsgefäßen, die obenseitig wannenartig ausgehöhlt sind, wobei die Behandlungseinrichtung eine Abgabevorrichtung mit Mitteln zur ortsselektiven Ablage von Flüssigkeitströpfchen auf den Reagenzträgerkörpern nach dem Inkjet-Druckprinzip umfasst, eine Transportvorrichtung zum Transportieren solcher Reagenzträgerkörper aufeinander folgend und in einem kontinuierlichen Durchlauf durch eine Behandlungszone der Behandlungseinrichtung hindurch, und eine Steuereinrichtung zur zeitlich abgestimmten Steuerung der Behandlungseinrichtung und der Transportvorrichtung, derart, dass die Behandlungseinrichtung an den die Behandlungszone kontinuierlich durchlaufenden Reagenzträgerkörpern jeweils wenigstens einen vorbestimmten Behandlungsschritt durch ortsselektive Ablage von Flüssigkeitströpfchen auf den Reagenträgerkörpern mittels der Abgabevorrichtung während der Transportbewegung der betreffenden Reagenzträgerkörper ausführt.

Bei der Präparationsvorrichtung weist die Behandlungseinrichtung vorzugsweise eine Abgabevorrichtung zur gesteuert dosierten Abgabe wenigstens einer Substanz, insbesondere Flüssigkeit, an die Reagenzträgerkörper auf. Die Abgabevorrichtung umfasst gemäß der erfindungsgemäßen Präparationsvorrichtung Mittel zur ortsselektiven Ablage von Flüssigkeitströpfchen auf den Reagenzträgerkörpern.

Die Behandlungseinrichtung kann zusätzlich oder alternativ eine Abführungsvorrichtung zur Abführung wenigstens einer Substanz, insbesondere Flüssigkeit, von den Reagenzträgerkörpern aufweisen. Durch Kombination einer Abgabevorrichtung und einer Abführungsvorrichtung besteht die Möglichkeit des simultanen Austausches von Flüssigkeiten an dem Reagenzträgerkörper.

Vorzugsweise umfasst die Behandlungseinrichtung auch eine Spülvorrichtung für Reagenzträgerkörper. Ferner kann die Behandlungseinrichtung eine Trocknungsvorrichtung umfassen.

Die Behandlungseinrichtung kann zusätzlich oder alternativ Bestrahlungsmittel und optische Überwachungsmittel für Reagenzträgerkörper in der Präparationsvorrichtung umfassen.

Ausführungsformen der Erfindung werden nachstehend unter Bezugnahme auf die Figuren näher erläutert.
- Fig. 1: zeigt in einer schematischen Darstellung einen Teilbereich einer Präparationsvorrichtung nach der Erfindung, wobei als Behandlungseinrichtung ein stationär gehaltener, multipler Tröpfchenerzeuger zum Einsatz kommt, durch dessen Behandlungszone hindurch aufeinander folgende Reagenzträgerkörper bewegt werden.
- Fig. 2: zeigt in einer schematischen Darstellung eine Präparationsvorrichtung nach der Erfindung, wobei sowohl die Reagenzträgerkörper als auch die Behandlungseinrichtung in einem Behandlungszonenbereich in Bewegung sind.

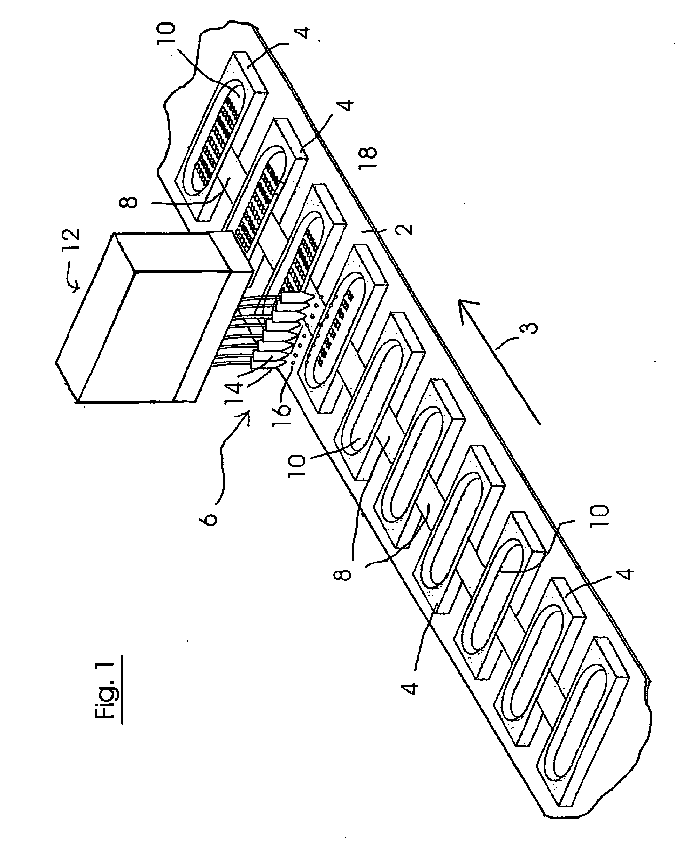

In Fig. 1 ist in perspektiver Darstellung ein Ausschnitt eines Förderbandes 2 erkennbar, welches Teil einer Transportvorrichtung für Reagenzträgerkörper 4 ist. Die Reagenzträgerkörper 4 sind vorgefertigte Reaktionsgefäße und können z.B. Spritzgussteile aus Polystyrol oder dgl. sein. Im Beispielsfall der Fig. 1 werden die einzelnen Reagenzträgerkörper 4 im Verband durch die Behandlungszone 6 mittels der Transportvorrichtung 2 transportiert, wobei die Reagenzträgerkörper 4 z.B. durch später leicht entfernbare Verbindungsstreifen 8 miteinander verbunden sind. In anderen Ausführungsformen der Erfindung können die vorgefertigten Reagenzträgerkörper 4 ohne Verbindungsstreifen und somit getrennt voneinander auf der Fördereinrichtung 2 transportiert werden.

An ihrer Oberseite sind die Reagenzträgerkörper 4 wannenartig ausgehöhlt, wobei der Wannenboden die zu behandelnde Oberfläche 10 des Reagenzträgerkörpers 4 aufweist. Die Außenabmessungen eines Reagenzträgerkörpers 4 betragen in einem Beispielsfall ca. 22 mm x 7 mm x 3 mm. Selbstverständlich sind andere Abmessungen und Gestaltungen der Reagenzträger möglich.

Als Behandlungseinrichtung ist in Fig. 1 ein nach dem Inkjet-Prinzip funktionierender und von einer (nicht gezeigten) elektronischen Steuereinheit gesteuerter Tröpfchenerzeuger 12 schematisch dargestellt. Der Tröpfchenerzeuger 12 dient dazu, auf der Fläche 10 der Reagenzträgerkörper 4 an vorbestimmten Positionen Flüssigkeitströpfchen abzulegen. In dem Beispielsfall der Fig. 1 umfasst der Tröpfchenerzeuger 12 acht Tröpfchenabgabedüsen 14, denen von Flüssigkeitsreservoirs bestimmte Flüssigkeiten zugeführt werden, die dann tröpfchenweise an die Reagenzträgerkörper 4 abgegeben werden. Die Tröpfchen 16 gelangen im Freiflug über eine Strecke von einigen Millimetern auf die Oberfläche 10 des jeweils die Behandlungszone 6 durchlaufenden Reagenzträgerkörpers 4. Vorzugsweise, aber nicht zwingend erforderlich erfolgen die kontinuierliche Transportbewegung der Reagenzträgerkörper 4 mit konstanter Geschwindigkeit und die Tropfenerzeugung des Tröpfchenerzeugers 12 mit konstanter Frequenz.

Überwachungsmittel der Steuereinrichtung sorgen dafür, dass die Tröpfchenabgabe des Tröpfchenerzeugers 12 und der Transport der Reagenzträgerkörper 4 so aufeinander abgestimmt sind, dass Tröpfchen 16 nur in der vorbestimmten Weise die Oberflächenbereiche 10 der Reagenzträgerkörper 4 erreichen können. Mit der Anordnung gemäß Fig. 1 können problemlos mehrere identische Bereiche (Replikate) mit hoher Taktrate hergestellt werden. Bei den bereits behandelten Reagenzträgerkörpern 4 in Fig. 1 sind Replikate mit gleichem Grauton dargestellt, wobei unterschiedliche Reaktanden in unterschiedlichem Grauton markiert sind. Die Reagenzträgerkörper 4 werden somit mit Bereichen unterschiedlicher Reaktivität im Assay präpariert.

Die Anordnung gemäß Fig. 1 ermöglicht bedarfsweise eine Prozessführung, bei der die Relativbewegung zwischen Reagenzträgerkörpern 4 und Tröpfchenerzeuger 12 mit konstanter Geschwindigkeit abläuft und die einzelnen Tröpfchengeneratoren (Düsen 14) mit zeitlich konstanter Frequenz, jedoch von Düse 14 zu Düse 14 individuell geringfügig unterschiedlicher Frequenz betrieben werden. Dann entstehen Muster mit individuellen Abständen zwischen jeweiligen Replikaten, was zur Identifikation des Tröpfchentyps bzw. des Analyttyps im Assay herangezogen werden kann.

Bei Testläufen wurden Reagenzträgerkörper 4 mit einem Vorschub von etwa 10 cm/s durch die Behandlungszone 6 hindurchgeführt. Die Tröpfchengeneratoren 14 wurden mit einer Frequenz von 500 Hz betrieben, wobei Reagenzträgerexemplare mit ca. 25 Replikaten je Flüssigkeitstyp präpariert wurden. Die erreichte Leistung liegt bei über 50 000 Exemplaren pro Stunde. Durch Optimierungsmaßnahmen ist die Ausbringleistung noch erheblich zu steigern.

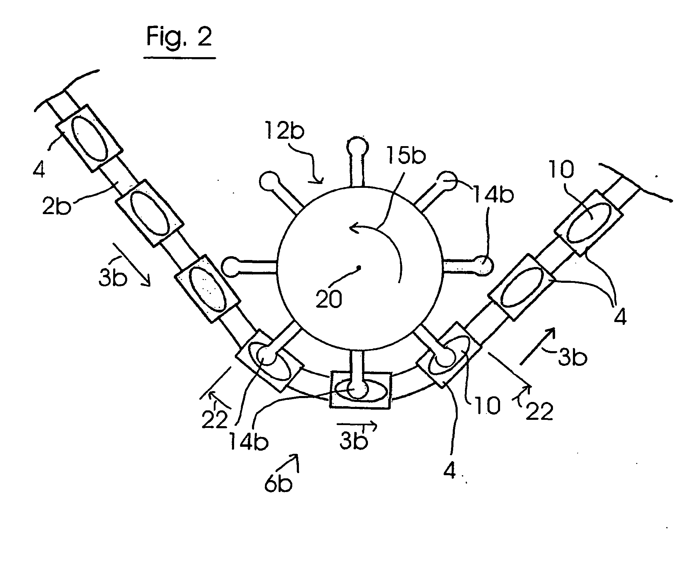

Fig. 2 zeigt in schematischer Draufsichtdarstellung ein weiteres Ausführungsbeispiel eines Aspekts der Erfindung, wobei dieser Aspekt die kontinuierliche Bewegung sowohl der Reagenzträgerkörper 4 als auch der Behandlungseinrichtung 12b während der Behandlung der Reagenzträgerkörper 4 betrifft.

Die Transportvorrichtung transportiert die Reagenzträgerkörper 4 der Reihe nach längs der Strecke 3b. Dabei durchlaufen die Reagenzträgerkörper 4 eine Behandlungszone 6b, in welcher sie mittels der Behandlungseinrichtung 12b behandelt werden. Die Behandlungseinrichtung 12b umfasst im Beispielsfall acht in Bezug auf ein gemeinsames Zentrum 20 radial nach außen abstehende und durch gleiche Winkelabstände voneinander separierte Behandlungsköpfe 14b, welche sich um das gemeinsame Zentrum 20 herum drehen. Die durch den Pfeil 15b angedeutete Drehrichtung der Behandlungseinrichtung 12b und deren Drehgeschwindigkeit sind unter der Kontrolle einer (nicht gezeigten) elektronischen Steuereinrichtung so auf die Bewegung der Reagenzträgerkörper 4 abgestimmt, dass die die Behandlungszone 6b durchlaufenden Reagenzträgerkörper 4 über einen vorbestimmten Winkelbereich 22 von einem jeweils zugeordneten Behandlungskopf 14b begleitet und dabei behandelt werden. Bei dem Beispiel gemäß Fig. 2 werden jeweils drei Reagenzträgerkörper 4 simultan während der gemeinsamen Bewegungen der Reagenzträgerkörper 4 und der Behandlungseinrichtung 12 behandelt. Die Behandlungseinrichtung 12b bei dem Ausführungsbeispiel nach Fig. 2 kann z.B. eine Flüssigkeitsabgabevorrichtung sein, welche über jeden der Behandlungsköpfe 14b in dosierter Weise Flüssigkeit an die Reagenzträgerkörper 4 abgeben kann. Bei sehr hohen Durchsatzgeschwindigkeiten kann es sinnvoll sein, den Auslösezeitpunkt so zu wählen, dass bereits Flüssigkeit ausgestoßen wird, noch bevor der betreffende Reagenzträgerkörper 4 unterhalb der Austrittsdüse positioniert ist.

Vorzugsweise kommen Überwachungsmittel der Steuereinrichtung zum Einsatz, um das Auslösen des Flüssigkeitstransfers mit der Bewegung der Reagenzträger synchronisieren zu können. Solche Überwachungsmittel können z.B. Lichtschranken, Bildaufnahmegeräte, wie etwa CCD-Sensoren, usw. sein.

Weiterhin kann es vorteilhaft sein, den Vorgang der Flüssigkeitszuführung zum Reagenzträgerkörper zu kaskadieren, so dass die Beaufschlagung des Reagenzträgerkörpers mit Flüssigkeit nicht in einer einzigen, sondern in mehreren Abgaben aus N Kanälen, die aus einem gemeinsamen Vorratsgefäß gespeist werden, erfolgt.

Bei dem in Fig. 2 skizzierten Prinzip kann es vorgesehen sein, dass sich die Behandlungsköpfe 14b beim Durchlaufen der Behandlungszone 6b mit anderer Geschwindigkeit bewegen als die Reagenzträgerkörper 4, so dass jeder Reagenzträgerkörper 4 in der Behandlungszone 6b von mehreren Behandlungsköpfen 14b nacheinander beaufschlagt werden kann, etwa um nacheinander einen Absaugschritt, einen Flüssigkeitszuführungsschritt und ggf. einen Bestrahlungsschritt unterzogen zu werden.

Die Funktionen und Aufbauten geeigneter Flüssigkeitsabgabevorrichtungen, Absaugvorrichtungen, Spül- und Waschvorrichtungen und Detektionsvorrichtungen sind dem Fachmann bekannt, so dass es in seinem Ermessen gestellt bleiben kann, eine oder mehrere solcher Vorrichtungen in der erfindungsgemäßen Weise in einer Präparationsvorrichtung der hier betrachteten Art einzusetzen und das Verfahren nach der Erfindung durchzuführen, um bei der Herstellung bzw. Präparation von bindungsfähigen Reagenzträgern Start- und Stoppvorgänge bei der Handhabung der Reagenzträgerkörper 4 möglichst weitgehend zu vermeiden.

Es sei noch darauf hingewiesen, dass die in den Figuren dargestellten Reagenzträgerkörper 4 nur Beispiele darstellen. Die Erfindung kann selbstverständlich auch unter Verwendung anders geformter Reagenzträgerkörper praktiziert werden.

## Patentansprüche

1. Verfahren zur Herstellung von bindungsfähigen und zur Bestimmung von Analyten in Flüssigkeiten geeigneten Reagenzträgern, wobei Reagenzträgerkörper (4) einer Behandlungseinrichtung (12; 12b) in einer Präparationsvorrichtung zugeführt und in einer Behandlungszone (6; 6b) der Behandlungseinrichtung (12; 12b) jeweils wenigstens einem Behandlungsschritt durch ortsselektive Ablage von Flüssigkeitströpfchen auf dem Reagenzträgerkörper (4) bei Durchführung einer Relativbewegung zwischen der Behandlungseinrichtung (12; 12b) und dem Reagenzträgerkörper (4) unterzogen werden, wobei die ortsselektive Ablage von Flüssigkeitströpfchen auf die Reagenzträgerkörper (4) mittels eines Tröpfchenerzeugers nach dem Inkjet-Druckprinzip erfolgt,
**dadurch gekennzeichnet,**
**dass** als Reagenzträgerkörper (4) vorgefertigte Reaktionsgefäße, die obenseitig wannenartig ausgehöhlt sind, verwendet und in einem kontinuierlichen Durchlauf durch die Behandlungszone (6; 6b) hindurchtransportiert werden und dass dabei der wenigstens eine Behandlungsschritt an den bewegten Reagenzträgerkörpern (4) erfolgt.

2. Verfahren zur Herstellung von Reagenzträgern nach Anspruch 1,
**dadurch gekennzeichnet, dass** bei Durchführung des Behandlungsschrittes sowohl der jeweilige Reagenzträgerkörper (4) als auch die Behandlungseinrichtung (12b) bewegt werden.

3. Verfahren zur Herstellung von Reagenzträgern nach Anspruch 1 oder 2,
**dadurch gekennzeichnet, dass** als weiterer Behandlungsschritt das Abführen wenigstens einer Substanz, insbesondere einer Flüssigkeit, von dem Reagenzträgerkörper (4) bei dessen kontinuierlicher Bewegung durch eine betreffende Behandlungszone durchgeführt wird.

4. Verfahren zur Herstellung von Reagenzträgern nach einem der vorhergehenden Ansprüche, **dadurch gekennzeichnet, dass** es zur Herstellung von
- streptavidinbeschichteten Trägern oder
- HBc-Antigen-Trägern oder
- Multianalyt-Trägern
angewandt wird.

5. Präparationsvorrichtung zur Durchführung des Verfahrens nach einem der Ansprüche 1 bis 4, **gekennzeichnet durch**
wenigstens eine Behandlungseinrichtung (12; 12b) zur Behandlung von Reagenzträgerkörpern (4) in Form von Reaktionsgefäßen, die obenseitig wannenartig ausgehöhlt sind, wobei die Behandlungseinrichtung eine Abgabevorrichtung (12) mit Mitteln zur ortsselektiven Ablage von Flüssigkeitströpfchen auf den Reagenzträgerkörpern (4) nach dem Inkjet-Druckprinzip umfasst,
eine Transportvorrichtung (2; 2b) zum Transportieren solcher Reagenzträgerkörper (4) aufeinander folgend und in einem kontinuierlichen Durchlauf **durch** eine Behandlungszone (6; 6b) der Behandlungseinrichtung (12; 12b) hindurch, und
eine Steuereinrichtung zur zeitlich abgestimmten Steuerung der Behandlungseinrichtung (12;12b) und der Transportvorrichtung (2; 2b), derart, dass die Behandlungseinrichtung (12; 12b) an den die Behandlungszone (6; 6b) kontinuierlich durchlaufenden Reagenzträgerkörpern (4) jeweils wenigstens einen vorbestimmten Behandlungsschritt **durch** ortsselektive Ablage von Flüssigkeitströpfchen auf den Reagenzträgerkörpern (4) mittels der Abgabevorrichtung während der Transportbewegung der betreffenden Reagenzträgerkörper (4) ausführt.

6. Präparationsvorrichtung nach Anspruch 5, **dadurch gekennzeichnet, dass** die Behandlungseinrichtung eine Abgabevorrichtung (12b) zur insbesondere gesteuert dosierten Abgabe wenigstens einer Substanz, insbesondere Flüssigkeit, an die Reagenzträgerkörper (4) während dessen Transportbewegung aufweist.

7. Präparationsvorrichtung nach einem der Ansprüche 5 bis 6, **dadurch gekennzeichnet, dass** die Behandlungseinrichtung eine Abführungsvorrichtung zur Abführung wenigstens einer Substanz, insbesondere Flüssigkeit, von den Reagenzträgerkörpern aufweist.

8. Präparationsvorrichtung nach einem der Ansprüche 5 bis 7, **dadurch gekennzeichnet, dass** die Behandlungseinrichtung eine Spülvorrichtung für Reagenzträgerkörper aufweist.

## Claims

1. Process for producing reagent carriers having a binding capability that are suitable for determining analytes in liquids, wherein reagent carrier bodies (4) are fed to a treatment device (12; 12b) in a preparation device and wherein said reagent carrier bodies (4) are subjected to at least one treatment step in a treatment zone (6; 6b) of the treatment device (12; 12b) by a locally-selective depositing fluid droplets on the reagent carrier body (4) during a relative movement between the treatment device (12; 12b) and the reagent carrier body (4), wherein said locally-selective deposition of fluid droplets on said reagent carrier body (4) is performed by means of a droplet generator according to the principle of inkjet printing,
**characterized in that**
prefabricated reaction vessels are used as reagent carrier bodies (4) and that said prefabricated reaction vessels are hollowed out at their top sides in a trough-shaped manner, wherein said reagent carrier bodies (4) are moved in a continuous passage through the treatment zone (6; 6b) and wherein said at least one treatment step is carried out on the moving reagent carrier bodies (4).

2. Process for producing reagent carriers as claimed in claim 1, **characterized in that** the respective reagent carrier body (4) as well as the treatment device (12b) are moved when the treatment step is carried out.

3. Process for producing reagent carriers as claimed in claim 1 or 2, **characterized in that** a further treatment step is performed wherein said further treatment step comprises removing at least one substance in particular a liquid from the reagent carrier body (4) during its continuous movement through a respective treatment zone.

4. Process for producing reagent carriers as claimed in one of the previous claims, **characterized in that** it is used to produce
- streptavidin-coated carriers or
- HBc antigen carriers or
- multianalyte carriers.

5. Prepraration device for carrying out the process as claimed in one of the claims 1 - 4, **characterized by**
at least one treatment device (12; 12b) for treating reagent carrier bodies (4) in the form of reaction vessels, which are hollowed out at the top-side in a trough-shaped manner, said treatment device comprising a dispensing device (12) having means for locally-selective depositing fluid droplets on said reagent carrier bodies (4) according to the principle of inkjet printing,
a transport device (2; 2b) for transporting such reagent carrier bodies (4) successively and in a continuous passage through a treatment zone (6, 6b) of the treatment device (12; 12b), and
a control device for the time-coordinated control of the treatment device (12; 12b) and the transport device (2; 2b) such that the treatment device (12; 12b) in each case performs at least one predetermined treatment step on the reagent carrier bodies (4) passing through the treatment zone (6; 6b) in a continuous manner, wherein in that predetermined treatment step fluid droplets are locally-selective deposited on said reagent carrier bodies (4) by means of said dispensing device during the transport movement of the respective reagent carrier body (4).

6. Preparation device according to claim 5, **characterized in that** said treatment device has a dispensing device (12b) for the in particular controlled metered release of at least one substance, in particular a liquid on to the reagent carrier bodies during their transport movement.

7. Preparation device according to one of the claims 5 to 6, **characterized in that** said treatment device has a removal device for removing at least one substance in particular a liquid from the reagent carrier bodies.

8. Preparation device according to one of the claims 5 to 7, **characterized in that** the treatment device has a rinsing device for reagent carrier bodies.

## Revendications

1. Procédé de fabrication de supports à réactifs à pouvoir de liaison et appropriés pour déterminer des analytes en liquides, sachant que des corps de support à réactifs (4) d'un système de traitement (12 ; 12b) sont amenés à un dispositif de préparation et sont soumis dans une zone de traitement (6 ; 6b) du système de traitement (12 ; 12b) respectivement au moins à une étape de traitement par dépôt localisé de gouttelettes de liquide sur le corps de support à réactifs (4) lors de la réalisation d'un mouvement relatif entre le système de traitement (12 ; 12b) et le corps de support à réactifs (4), sachant que le dépôt localisé de gouttelettes de liquide sur le corps de support (4) à réactifs est effectué au moyen d'un générateur de gouttelettes selon le principe d'impression par jet d'encre,
**caractérisé en ce**
**qu'**on utilise comme corps de support à réactifs (4) des réacteurs préfabriqués creusés côté supérieur à la manière d'une cuve, lesquels réacteurs sont transportés à travers la zone de traitement (6 ; 6b) lors d'un cycle continu, et en ce que dans ce cadre l'étape de traitement au moins au nombre de une est effectuée au niveau des corps de support à réactifs (4) déplacés.

2. Procédé de fabrication de supports à réactifs selon la revendication 1,
**caractérisé en ce qu'**aussi bien le corps de support à réactifs (4) respectif que le système de traitement (12b) sont déplacés lors de la réalisation de l'étape de traitement.

3. Procédé de fabrication de supports à réactifs selon la revendication 1 ou 2,
**caractérisé en ce que** l'évacuation d'au moins une substance, en particulier d'un liquide, du corps de support à réactifs (4) est effectuée par une zone de traitement correspondante en tant qu'autre étape de traitement lors du déplacement en continu dudit corps de support à réactifs.

4. Procédé de fabrication de supports à réactifs selon l'une quelconque des revendications précédentes, **caractérisé en ce que** ledit procédé est appliqué pour la fabrication
- de supports recouverts de streptavidine ou ;
- de supports d'antigène HBc ou ;
- de supports de plusieurs analytes.

5. Dispositif de préparation servant à mettre en oeuvre le procédé selon l'une quelconque des revendications 1 à 4, **caractérisé par**
au moins un système de traitement (12 ; 12b) servant à traiter des corps de support à réactifs (4) sous la forme de réacteurs creusés côté supérieur à la manière d'une cuve, sachant que le système de traitement comporte un dispositif de distribution (12) doté de moyens servant à déposer de manière localisée des gouttelettes de liquide sur les corps de support à réactifs (4) selon le principe d'impression à jet d'encre,
un dispositif de transport (2 ; 2b) servant à transporter de tels corps de support à réactifs (4) les uns à la suite des autres et à travers une zone de traitement (6 ; 6b) du système de traitement (12 ; 12b) lors d'un cycle continu, et
un système de commande servant à commander de manière synchronisée le système de traitement (12 ; 12b) et le dispositif de transport (2 ; 2b) de telle manière que le système de traitement (12 ; 12b) réalise au niveau des corps de support à réactifs (4) traversant en continu la zone de traitement (6 ; 6b) respectivement au moins une étape de traitement prédéfinie par dépôt localisé de gouttelettes de liquide sur les corps de support à réactifs (4) au moyen du dispositif de distribution pendant le mouvement de transport des corps de support à réactifs (4) concernés.

6. Dispositif de préparation selon la revendication 5, **caractérisé en ce que** le système de traitement présente un dispositif de distribution (12b) servant en particulier à distribuer de manière dosée et de manière commandée au moins une substance, en particulier un liquide, au corps de support à réactifs (4) pendant son mouvement de transport.

7. Dispositif de préparation selon l'une quelconque des revendications 5 à 6, **caractérisé en ce que** le système de traitement présente un dispositif d'évacuation servant à évacuer au moins une substance, en particulier un liquide, des corps de support à réactifs.

8. Dispositif de préparation selon l'une quelconque des revendications 5 à 7, **caractérisé en ce que** le système de traitement présente un dispositif de rinçage pour des corps de support à réactifs.
